# EUROPEAN PATENT SPECIFICATION

(11) **EP 1 755 560 B1**
(45) Date of publication and mention of the grant of the patent: **11.04.2012**
(21) Application number: 05754171.6
(22) Date of filing: 23.05.2005
(51) Int. Cl.: A61K 9/20

(54) **PHARMACEUTICAL TABLETS HAVING A RELATIVELY INACTIVE SEGMENT**
PHARMAZEUTISCHE TABLETTEN MIT EINEM RELATIV INAKTIVEN SEGMENT
COMPRIMES PHARMACEUTIQUES POSSEDANT UN SEGMENT RELATIVEMENT INACTIF

(30) Priority: 21.05.2004 US 573042 P; 21.05.2004 US 573134 P
(43) Date of publication of application: 28.02.2007
(73) Proprietor: Accu-Break Technologies, Inc., Plantation, FL 33324 (US)
(72) Inventor: SOLOMON, Lawrence, Boca Raton, FL 33433 (US); KAPLAN, Allan, S., Boaca Raton, FL 33433 (US)
(74) Representative: Coletti, Raimondo
(86) International application number: PCT/US2005/018639
(87) International publication number: WO 2005/112898

(56) References cited:
- US-A- 3 336 200
- US-A- 4 258 027
- US-A- 4 905 589
- US-A- 5 738 874
- US-A- 5 756 124
- US-B1- 6 602 521
- DATABASE WPI Week 199410 Derwent Publications Ltd., London, GB; AN 1994-077223 XP002293384 -& JP 06 009375 A (TAKEDA CHEM IND LTD) 18 January 1994 (1994-01-18)
- VAN SANTEN E ET AL: "Breaking of scored tablets: a review" EUROPEAN JOURNAL OF PHARMACEUTICS AND BIOPHARMACEUTICS, ELSEVIER SCIENCE PUBLISHERS B.V., AMSTERDAM, NL, vol. 53, no. 2, March 2002 (2002-03), pages 139-145, XP004342806 ISSN: 0939-6411

## Description

### FIELD OF THE INVENTION

The invention involves layered immediate release pharmaceutical tablets comprising a layer containing a drug and a layer either lacking a drug or containing said drug as part of a different granulation, such as in a less concentrated form.

### BACKGROUND

The invention derives from the need to solve two related problems within the pharmaceutical industry. One is that immediate release pharmaceutical tablets are commonly broken, and are often produced with a score to aid breaking, but that tablet breaking is well-documented to suffer many problems, some of which are delineated below. The second problem is that combination drug products are typically produced as homogeneous mixtures or as capsules, so that a physician prescribing these products, such as to treat arterial hypertension, is limited in adjusting the dose of only one of said combination.

Documentation of the importance of the first problem is summarized as follows:
Many drugs require dosage adjustments, such as warfarin, the scored tablets of which are frequently broken. These dosage adjustments through tablet breaking by patients have been determined to be imprecise. As the following discussion demonstrates, for many years experts have called upon the pharmaceutical industry to improve the quality of tablet breaking, yet such has not been optimized until the current invention.

In 1984, Stimpel et al. ("Stimpel"), described the relative accuracy of breaking of various tablets for treatment of cardiovascular problems. M. Stimpel et al., "Breaking Tablets in Half." The Lancet (1984) :1299. Even though breaking was performed by a sophisticated, dexterous person, Stimpel found that breaking was not accurate, and opined that real world use by patients wold provide yet more unsatisfactory results. Stimpel called upon the pharmaceutical industry to improve the accuracy of splitting tablets: "Clearly any assumption that halving a tablet will not lead to inaccurate doses is invalid. This potential source of inaccuracy could be even more significant in clinical situations (our study was done under ideal conditions) and the pharmaceutical industry should tackle it, either by improving divisibility (as already has been done for lopressor and logroton) or, even better, by marketing a wider range of unscored tablets to provide all the doses that might be indicated clinically."

Despite that finding and statement, and despite the issuance of various patents relating to optimizing a scoring pattern and/or tablet shape, Rodenhuis et al., (2004) noted that: "Improving the functioning of score lines may be a more practical approach than banning this dosage form" (emphasis added). N. Rodenhuis et al., "The rationale of scored tablets as dosage form." European J. of Pharmaceutical Sciences 21 (2004):305-308 (hereafter "Rodenhuis"). Rodenhuis observed that European regulatory authorities started a policy to discourage scoring of tablets in 1998. This policy change, according to Rodenhuis, likely related to "many recent reports of bad functioning score lines," that "many scored tablets are difficult to break," and that "many scored tablets show unsatisfactory mass uniformity of the subdivided halves." The authors then go on to describe useful aspects of scoring tablets. For a comprehensive review article on this topic, see van Santen, E., Barends, D.M. and Frijlink, H.W. "Breaking of scored tablets: a review." European J. of Pharmaceutics and Biopharmaceutics 53 (2002):139-145.

Some current studies that demonstrate the severity of the problem are described below.
Peek et al., (2002), studied tablet splitting by "elderly patients" aged 50-79. Peek, B.T., A1-Achi, A., Coombs, S.J. "Accuracy of Tablet Splitting by Elderly Patients." The Journal of the American Medical Association 288 No.4 (2002):139-145. Breaking scored tablets with mechanical tablet splitters without specific instruction led to highly unsatisfactory separating of the tablets. For example, warfarin 5 mg was on average split into 1.9 and 3.1 mg tablets. This potent anticoagulant has such a narrow therapeutic range that 2, 2.5, and 3 mg tablet doses are manufactured Biron et al., (1999), demonstrated that warfarin 10 mg also often split to less than 4.25 or greater than 5.75 mg. Biron, C., Liczner, P., Hansel, S., Schved, J.F., "Oral Anticoagulant Drugs: Do Not Cut Tablets in Quarters." Thromb Haemost 1201 (1999). In addition, they demonstrated that loss of mass due to crumbling or chipping of the breaking of the warfarin tablets was statistically significant. They also demonstrated that quartering of the tablets was grossly inaccurate.

McDevitt et al., (1998), found that 25 mg unscored hydrochlorothiazide (HCTZ) tablets were manually split badly enough that 12.4% deviated by more than 20% from ideal weight. McDevitt, J.T., Gurst, A.H., Chen, Y. "Accuracy of Tablet Splitting." Pharmacotherapy 18 No.1(1998):193-197. 77% of the test subjects stated that they would be willing to pay a premium for individually produced HCTZ 12.5 mg tablets rather than split unscored 25 mg tablets.

Rosenberg et al., (2002), studied pharmacist-dispensed split tablets. Rosenberg, J.M., Nathan, J.P., Plakogiannis, F. "Weight Variability of Pharmacist-Dispensed Split Tablets." Journal of American Pharmaceutical Association 42 No.2 (2002):200-205. They found that "tablet splitting resulted in an unacceptably high incidence of weight variation." They recommended that "standards should be developed to ensure uniformity of split tablets."

Teng et al., (2002), using a trained individual in a laboratory setting to split tablets, concluded that "the majority of the 11 drug products we tested, when assessed for their ability to be split into half-tablets of equal dose, failed a liberally interpreted USP (United States Pharmacopeia) uniformity test... The practice of dividing tablets to save costs or to improve a dosage regimen ... is not recommended for patients using drugs with more substantial toxicity and steep dose-response efficacy curves." Teng, J., Song, C.K., Williams, R.L., Polli, J.E. "Lack of Medication Dose Uniformity in Commonly Split Tablets." Journal of American Pharmaceutical Association 42 No. 2 (2002):195-199.

Rodenhuis reported that 31% of all tablets in one Netherlands study were subdivided before being swallowed. In the U.S., many "managed care" insurance organizations encourage tablet splitting by patients including of unscored, irregularly-shaped tablets. Many drug products in the US are unscored or are provided as capsules despite being tablettable. The invention herein provides a means of ameliorating the above problems.

The second problem has, for example, hindered the acceptability of combination treatments for arterial hypertension ("hypertension"). If there were no cost advantage to a patient in taking one dosage form comprising two drugs (a "combination" dosage form) as opposed to two dosage forms each comprising one drug, and if the patient were as willing and able to take two dosage forms as to take one, there would thus be a disadvantage to a combination dosage form, due to the inflexibility of dealing with changing circumstances such as fluctuating blood pressure or the appearance of side effects to a drug. This observation, in the U.S., has been propounded many times over the years, but combination treatments for hypertension have proven popular for cost and potential compliance reasons, nonetheless.

In both of the above cases, the present invention provides a novel and optimal means of ameliorating both of the above problems.

The United States patent US5738874 discloses controlled release tablets, intended to provide different release rates from different layers.

The United States patent US3336200 describes immediate release tablets formed only by a single layer, having homogenous compositions, and it describes layered (segmented) tablets containing a controlled release layer, thus being controlled release tablets.

The United States patents US5756124 and US4258027 relate to single layer, homogenous compositions tablets.

The United States patent US6602521 describes tablets having immediate release and extended release compositions to provide two different release profiles for the active drugs contained in each tablet compartment, thus being a controlled release product.

The Japanese patent document JP06009375 (Database WPI AN 1994-077223) discloses dividable tablets that are formed by adhering together two subunits with adhesive or cement layer.

The prior art as it relates to immediate release pharmaceutical tablets comprising one drug, or a mixture of a plurality of drugs within one granulation, appears straightforward: it has not been taught to place two substantially identical layers in different places of an immediate release pharmaceutical tablet with an interposed different layer, or to utilize a drug in two different concentrations in the same tablet. The current invention explains reasons why both of the above may be useful.

The prior art regarding combination drugs teaches separating them into different layers with an interposed inactive layer (i.e., one derived from a granulation lacking drug) only when there is a physical or chemical incompatibility between them. In that case, the prior art specifically teaches that said "separating layer" be of as limited a size as is necessary to separate the incompatible layers. In contrast, the present invention teaches placing compatible granulations in different parts of the tablet, with an interposed preferably inactive layer (preferably of adequate height to allow it to be broken through); or, in the case of incompatible substances, requiring that any separating layer be of a height different than the current teaching regarding layers comprising incompatible substances.

### SUMMARY OF THE INVENTION

The invention provides an immediate release pharmaceutical tablet obtainable by production on a layer press and comprising vertically disposed segments wherein the drug-containing segments are separated by an inner segment that lacks a pharmacologically effective quantity of drug and the height of which is great enough to allow said inner segment to serve as breaking region, said tablet containing a drug or drugs, in which:
(a) said tablet includes at least one segment created from an inactive granulation and all segments that contain a pharmacologically effective dose of a drug or drug contain the same drug or combination of drugs at the same ratio;
(b) said tablet includes: (i) a first segment containing a drug or drugs; (ii) a third segment containing a drug or plurality of drugs which is/ are different from the drug or drugs in said first segment, and (iii) a second segment that is interposed between said first and third segments and that has an undetectable amount of, or else a pharmacologically ineffective amount of, any drug present in said tablet; (iv) physical and chemical compatibility between said first and third segment;
   or,
(c) said tablet includes: (i) a first segment containing a drug or two or more drugs; (ii) a third segment containing a drugs or drugs which are different from the drug or drugs in said first segment; (iii) a second segment that is interposed between said first and said third segment and that has an undetectable amount of, or a pharmacologically ineffective amount of, any drug present in said tablet; and, (iv) said third segment has either or both of the following: a height of at least 2.5 mm or an effective height of at least 1.5 mm.

The preferred core tablets of the invention are compressed tablets having at least two compositionally distinct segments, with a first segment containing an active drug and a second segment that:
(b) has no detectable drug or the same drug which is in said first segment in a pharmacologically ineffective amount, and said tablet also includes a third segment having the same drug that is present in said first segment; or
(d) has no detectable drug or the same drug which is in said first segment in a pharmacologically ineffective amount, and said tablet also includes a third segment having a different drug from the drug that is present in said first segment wherein the components of said third segment are compatible with the components of said first segment; or
(e) has no detectable drug or the same drug which is in said first segment in a pharmacologically ineffective amount, and said tablet also includes a third segment having a different drug from the drug that is present in said first segment, said second segment having a vertical height of at least 3mm;

The invention involves immediate release pharmaceutical tablets. The terms "active agent," "active drug," "drug," "active pharmaceutical ingredient" and "pharmacologically active agent" may be used interchangeably herein to refer to a chemical material or compound which, when administered to an organism (human or animal) induces a pharmacologic effect, and which includes prescription and non-prescription pharmaceutical compounds; and such substances as pharmacologically effective doses of vitamins or cofactors and the like. Excluded as being drugs herein are such substances as foodstuffs and vitamins in recommended daily allowance guantities.

Tablets of the invention belong to the class of immediate release pharmaceutical tablets formulated with two or more layers, and are thus non-homogeneous. Tablets of the invention are adapted to be useful not only as whole tablets but also to be breakable into subunits known herein as tablettes, with accurate dosing both as whole tablets and in tablette form. The invention achieves these ends by utilizing in most of its preferred embodiments a segment that is created from a granulation free of active drug (an "inactive granulation").

It is a primary object of the invention to create immediate release pharmaceutical tablets adapted to be broken when it is desired to create a lower dosage (including a dosage of zero) of a drug or drugs present in the whole tablet, by allowing breaking through a segment containing no drug, or else either a minimal concentration of drug or mixture of drugs (on a w/w basis) or a pharmacologically ineffective quantity of drug or mixture of drugs, and less preferably through a segment that contains a more than minimal but decreased concentration of a drug relative to another segment in the tablet.

It is a primary object of the invention to apply the invention both to accurate dosing of single agent products and to combination products.

With regard to combination products, relevant introductory points are: A mixture of drugs within one granulation acts as a single drug in a granulation from the standpoint of the separability of one segment from another or from a portion of another per the invention, in that said two admixed drugs are inseparable one from another. On the other hand, the invention is very useful in the case that a given drug, or mixture of drugs, is present in a pharmacologically effective quantity in one segment, and a different drug or different mixture of drugs, are present in different segments. In a preferred embodiment in which, for example Drug A is present in a therapeutically effective quantity in an upper segment, an inner segment that lacks a pharmacologically effective quantity of any drug is interposed between two outer (i.e., top and bottom) segments, and Drug B is present in a therapeutically effective quantity in a lower segment, then the invention is most useful in the situation that the height and especially the "effective height" of said inner segment is great enough to allow said inner segment to serve as the breaking region of said tablet substantially without breaking through either outer segment. The prior art, however, is such that no minimum height of said inner segment is necessary for novelty of the invention if, in said tablet, all ingredients of the upper and lower segments are physically and chemically compatible with each other, because in immediate release tablets, no "separating" layer or segment has been utilized or taught to be utilized in such a case. In the specialized situation in which there is an incompatibility between components of said outer segments, then the prior art is such that any inner "separating" segment be limited in height to the minimum needed to eliminate the presence of any of said incompatibilities, for such reasons as to minimize the size of the tablet as a whole. In that case, the invention remains novel in any of its more preferred forms, in which there is an excess of said inner separating segment to allow it to be broken.

### BRIEF DESCRIPTION OF THE DRAWINGS

Fig. 1a is a cross-section of a taller than wide tablet looking towards the side of the tablet having a score;
Fig. 1b is a cross-section of the tablet of Fig. 1a looking at the side of the tablet where the score ends;
Figs. 2a-d are views of Fig 1a and Fig. 1b respectively when the tablets have been broken through the score;
Fig. 3 is a cross-section of a taller than wide tablet having two segments, one of which is about three-quarters of the length of the tablet;
Figs. 4a-b are views of Fig. 3 when the tablet has been broken at the approximate mid-point of the tablet;
Fig. 5 is a cross-section of a taller than wide tablet having five segments;
Figs. 6a-b are views of Fig. 5 when the tablet has been broken through one segment;
Figs. 7a-c are views of Fig. 5 when the tablet has effectively been broken through two segments in two steps, first by breaking the tablet and then by breaking the tablette of Fig. 6b;
Fig. 8 is a cross-section of a tablet having three segments. Fig. 9 is a perspective view of a scored tablet according to the invention;
Fig. 10 is a front view of a banded capsule according to the invention;
Fig. 11 is a front view of a tablet having perforations in the surface according to the invention; and
Fig. 12 is a front view of a tablet having two printed dotted on the surface according to the invention.

### DETAILED DESCRIPTION OF THE INVENTION

Tablets of the invention are preferably produced on a layer press, such as a tri-layer or five-layer high speed press manufactured by Korsch AG of Germany.
The tablets are formed from at least two different granulations; more preferred tablets comprise three vertically disposed segments.

Of the many tablets than can be produced according to the invention, some examples are:
A first granulation comprising amlodipine besylate (or, "amlodipine") enters into a die at a first filling station; a second granulation comprising inactive excipients enters on top of said first granulation at a second filling station; a granulation substantially identical in composition and quantity (weight) to said first granulation enters at a third filling station. After final compression, said tablet is ejected from the die. Each granulation, upon full entry into the die and thereafter, forms a layer. Ideally there is minimal, inadvertent mixing between different granulations in the formation of layers, but some mixing is to be expected and does not alter the improvement in the art of creating breakable tablets from the invention. Different granulations may be of the same or different colors.

By convention herein, the term "segments" may be used in place of "layers" in general in discussing the finished tablets of the invention, for reasons that are explained below.

A segment represents the entirety of a substantially homogeneous contiguous part of a tablet. A segment may be formed from more than one layer, however: If two substantially identical granulations entered the tablet die successively, with the second entering directly after and onto the first, such as at two successive filling stations during automated high-speed tablet manufacture, then the two granulations would each form a separate layer after entering, but when compressed, they would comprise one segment. A segment therefore is a basic unit of how the tablets of the invention prove useful. If, however, two different active drugs, such as different active drugs or different salts of the same active drug, were compressed one on top of the other, they would form two segments. Granulations comprising the same active drug but with dissimilar excipients would also two segments if one granulation were compressed onto another.

A segment formed by a plurality of layers that are formed from substantially identical granulations is called a compound segment. Compound segments may prove useful in situations of relatively large quantities of an inactive granulation, or granulation containing a drug or drugs, so that two consecutive fills ("feeds") of substantially identical granulation occur.

Probably the more common situation in tablets of the invention involves a layer formed from a granulation that is neither disposed upon nor under (i.e., does not adjoin and is not contiguous with) a substantially identical granulation. In this case, a simple segment is formed. In other words, a non-compound segment is a simple segment.

As used herein, such terms as "horizontal" ("transverse") and "vertical" when used in relation to a tablet, are based on the spatial orientation of the tablet as, and after, it is produced in a die, but before removal or ejection from the die. Current methods of manufacture produce tablets with one granulation entering the die on top of another, so that tablets of the invention produced in such a manner comprise one or more top (outer) segments, one or more bottom (outer) segments, and optionally one or more middle (inner) segments. A segment that is not a top or bottom (i.e., outer) segment is considered to be an inner segment. Of course, the lateral parts of an inner segment have an external surface.

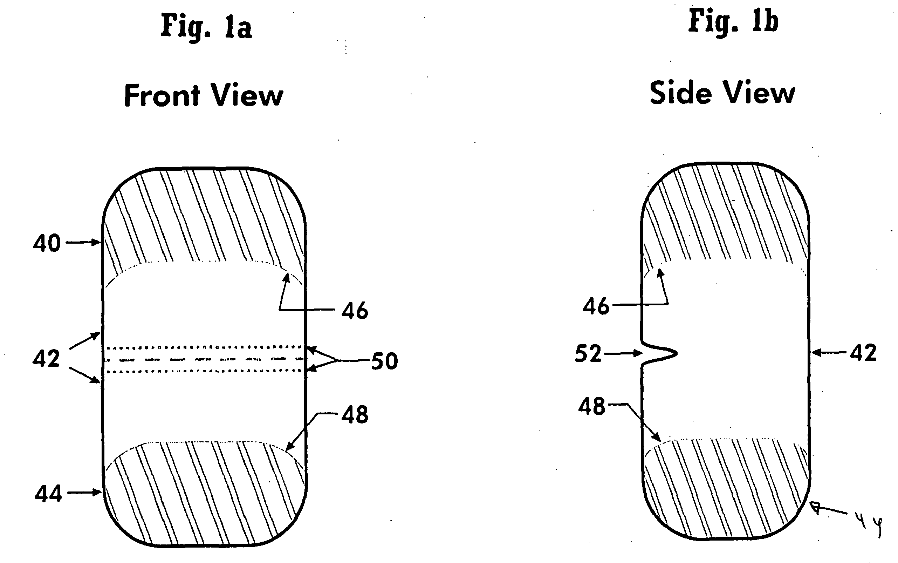

If separate granulations were to be sequentially placed in a die horizontally (side-to-side) and not vertically as is currently the practice, then the tablets so produced would be within the scope of the present invention as the same product would be produced. When the tablet of Fig. 1, for example, is laid on a flat table, it will tend to lie lengthwise at right angles to the manner in which it is formed in the die, so that if the three segments were all different colors, then the segments would appear to be arranged not vertically (one on top of the other), but rather horizontally (side-to-side). For consistency of terminology, such segments nonetheless are considered herein to be disposed vertically on top of each other.

Tablets of the invention are not formed using a cement, glue, adhesive, or the like, and are preferably uncoated.

The term "relatively inactive segment" refers to a segment that either contains an undetectable amount of any drug or contains a decreased concentration of any drug or drugs contained in another segment or segments in a pharmacologically effective quantity. By "decreased concentration" we mean that the concentration of a drug or drugs in said relatively inactive segment is no more than 80% that of said drug or drugs in another segment, more preferably no more than 20% of said other segment's drug or drugs concentration; most preferably said ratio is no more than 5%, however. The concentration of a drug or drugs in a segment means, herein, the ratio, on a weight to weight basis, of the drug or drugs in said segment to the total weight of said segment, which includes said drug or drugs and inactive excipients.

The tablets of the invention are best broken transversely in order to realize their benefits. They may be broken in standard ways, according to the invention such as either by applying force such as a cutting edge directly to the separation mark desired breaking region, or to other areas of the tablet, such as the outer segments, to cause the tablet to break at the desired location.

The drawings depict vertical cross-sectional views of tablets and tablettes of the invention. Tablets are depicted as if they were in the die, so that the top of the tablet as it is oriented on the page corresponds with the top of the tablet in the die. In other words, the top segment of the tablet as viewed contains the last granulation to enter the die. Tablettes are depicted as they would have been in the die as part of the tablet of which they were once parts.

Separation marks are intended to guide optional tablet breaking in the usual manner that is well known with scores, so that force will be applied to break the tablet at or about the separation mark in a direction that is substantially perpendicular to the surface on which it is desired that breakage of the tablet will be initiated. The tablet may be broken according to the invention either by applying force such as a cutting edge directly to the separation mark, or to other areas of the tablet, such as the outer segments, to cause the tablet to break at or about the separation mark and in the direction of the separation mark.

The separation mark or marks may comprise one or more of the following:
(a) a score in a side wherein said score is not oriented vertically ;
(b) indicia on at least side that locates aa desired breaking region of said tablet;
(c) a band which is located on one segment which is located on one segment or at an interface of te two segments; or
(d) a core of said tablet in which a first lower and a second upper segment have the same color and contain either the same drug in a pharmacologically effective quantity or both lack a pharmacologically effective quantity of any drug, and a third inner, interposed segment that has a different color from said first segment and has either the same drug as said first segment when said first segment has a pharmacologically effective quantity of a drug or has no pharmacologically effective quantity of a drug when said first segment lacks a pharmacologically effective quantity of any drug.

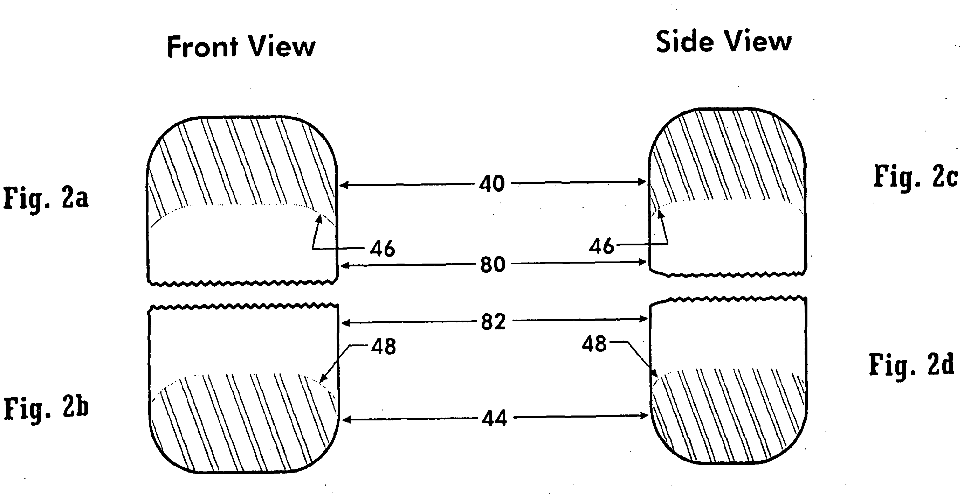

"Front views" refer to a cross-sectional view of a tablet that has a theoretical geometric plane passed through the tablet relative to a side which is arbitrarily designated as the front. Figures labeled as "side view", which also have a corresponding "front view" are taken as a cross-section through the whole tablet from the right side of a front view i.e. a side view is a cross-section that is taken by passing a plane through the vertical axis of the whole tablet at a 90° angle to the cross-sectional front view. Each front view represents a schematic cross-section that passes through the midpoint of the horizontal cross-section as measured from the front of the tablet to the back of the tablet or tablette. The front view is also parallel to the major axis of the tablet (e.g, for a tablet with a rectangular (but not square) transverse cross-section, the longer side of the perimeter is parallel with the plane that depicts the cross-sectional, front view. That plane is located half-way between the front and back surfaces of said tablet. The side views of Figs. 1b and 2c-d are taken from a vertically-oriented plane that passes through the midpoint of the longer transverse dimension (i.e., the width), and thus are located at and perpendicular to the mid-point of the front view. Drawings are of tablets that have a rectangular but not square horizontal cross-section at the vertical mid-point of the tablet.

Segments containing pharmacologically active amounts of a drug or drugs are shown crosshatched; pharmacologically ineffective segments are shown plain (clear, without crosshatching or stippling). The upper part of each figure corresponds to the upper part of a tablet, all of which are depicted as they are situated within a die after final compression and before ejection from the die. For consistency, tablettes are depicted in the same orientation as the tablets from which they are formed, although tablettes are created after tablet ejection from the die. Dotted lines in the tablets depicted in the figures may represent printed marks or other indicia, or scores that are present on or in the surface of the tablet and, if they represent a score, said score does not extend deeply enough into the tablet to appear in the cross-sectional front view. The transverse dotted lines reflecting scores shown in the Figures imply no intention to limit the depth of any scores of the tablets of the invention. Horizontal dotted lines on the front views that represent the surface scores are schematic, and do not necessarily represent the full vertical extent of a score, printed mark, or the like.

Tablettes are depicted with broken surfaces as indicated by a fine saw-tooth pattern. Such saw-tooth depiction is schematic and not intended to represent the actual pattern of breaking of a tablet (or tablette, which often leads to irregular edges even if said tablet is broken through a score.

Separation marks in the tablets depicted in the Figures are depicted as scores that are present on or in the surface of the tablet and that do not extend deeply enough into the tablet to appear in the cross-sectional front views are depicted in the drawings as dotted lines to reflect the location of said scores on or in the surface of the tablet (not shown). It is to be understood that the depth of a separation mark or other score may be deeper than one-half the widest cross-section of the tablet in a particular embodiment, and thus the transverse dotted lines reflecting scores that are separation marks shown in the Figures imply no intention to limit the depth of any scores of the tablets of the invention. Similarly, the tablets shown that contain scores do not limit the width or extent of said scores. The horizontal dotted lines on the front views that represent the surface scores are schematic, and do not necessarily represent the full vertical extent of the score. (Perforations or discontinuous scores through the width or depth of the tablets are not depicted herein, but remain within the scope of the invention, as are other marks on or physical changes to the tablet that create a separation mark.) Any scores or printed indicia that serve as separation marks are for convenience herein assumed to be on the front surface of the tablet, which is arbitrarily chosen from a vertically-oriented surface of the tablets.

The "side view" of a tablet is a cross-sectional view of the tablet rotated 90 degrees from the front view, and is shown in Fig. 2c and 2d. No dimension of the separation marks is limited by their depiction as dotted lines in any figure. Tablettes are depicted with broken surfaces as indicated by a fine saw-tooth pattern. Such saw-tooth depiction is schematic and not intended to represent the actual pattern of breaking of a tablet or tablette.

Figures 1a and 1b depict a tablet with compositionally substantially identical upper segment 40 and lower segment 44. Inner segment 42 contains trace amounts of the drug that is present in a therapeutically effective quantity in each of segments 40 and 44. Interfaces 46 and 48 represent regions in which the upper part of segment 42 and the lower part of segment 42 respectively adjoin upper segment 40 and lower segment 44. The curved interfaces result from the profile of the upper tablet punch which is curved. Score 52 is depicted in Fig. 1b. Dotted line 50 in Fig. 1a is a reflection of score 52 on the surface of the tablet (not shown), that does not penetrate half-way through the shorter transverse axis of the tablet.

Figs. 2a-d depicts tablettes formed from breaking the tablet of Figs. 1a and 1b through score 52. Inner segment 42 of Fig. 1a no longer exists as an intact segment. The upper tablette of Figs. 2a and 2c contains segment 80 that adjoins an intact upper segment 40 and the lower tablette contains segment 82 and intact segment 44.

Breaking the tablet of Fig. 1a and 1b through the score placed in segment 42 is clearly easier than breaking the tablet through its vertical dimension, which is currently the practice with scored layered (segmented) tablets. The fact that no break is made in the parts of the tablet where the active drug has been place provides for exceptionally accurate breaking relative to the active drug or drugs contained in the tablet.

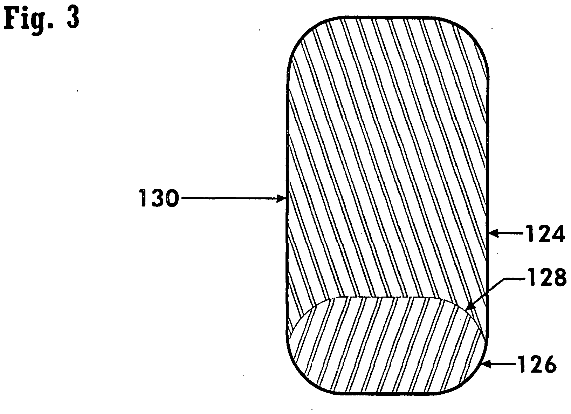

Fig. 3 demonstrates a two-segment tablet, each segment formed from a granulation containing a pharmacologically effective amount of medication. Upper (outer) segment 124 is larger than lower (outer) segment 126. Interface 128 indicates a region at which said segments are contiguous. A printed mark on the outer surface of the tablet (not shown) indicates a desired breaking point, as indicated by the location of arrow 130 that reflects the position of said surface printed mark. The two segments also have different colors, however, further allowing identification of which part of the tablet contains which segment.

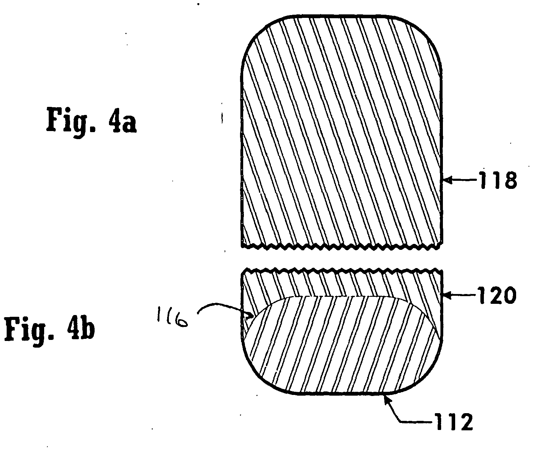

Figs. 4a and 4b depict the two tablettes formed by breaking the tablet of Fig. 3. The tablette of Fig. 4a consists of segment 118, which represents the bulk of segment 124 of Fig. 3. The tablette depicted in Fig. 4b contains segment 112 in an intact form and segment 120, which represents a less than half-portion of segment 126 of Fig. 3. Interface 116 indicates a region at which said segments are contiguous. The curved face is due to the profile of the tablet punch.

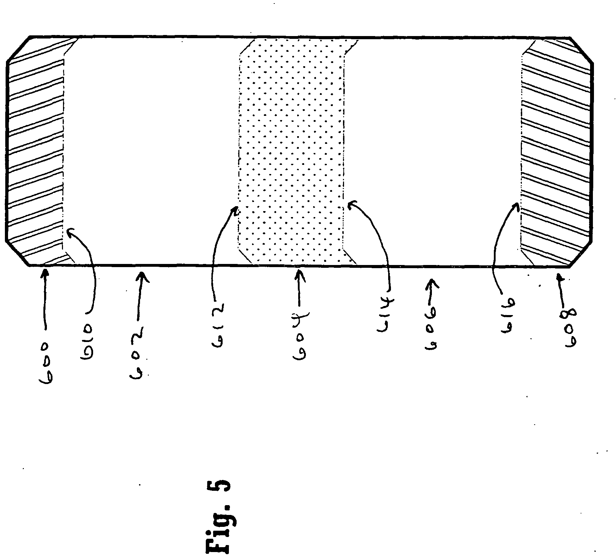

Fig. 5 illustrates a tablet more elongated than those previously demonstrated. This tablet is adapted, even more than the others, for ease of breaking through one segment.

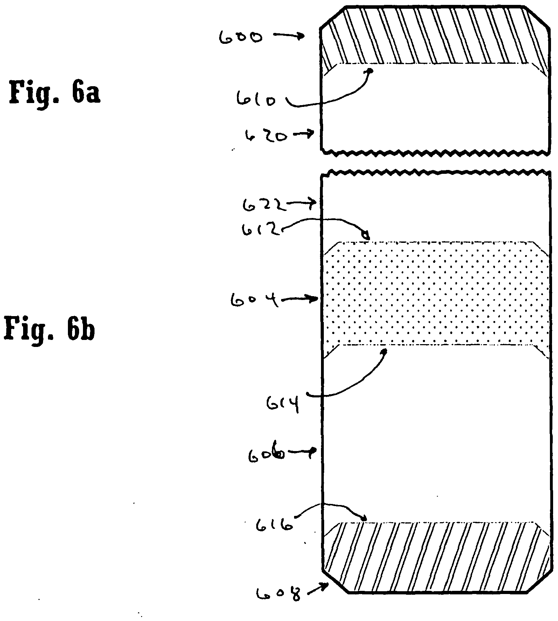

Upper segment 600 is provided with a therapeutic quantity of a drug; stippled inner segment 604 is provided with a therapeutic quantity of a different drug; and, lower segment 608 is provided with a therapeutic quantity of a drug different from that found in a therapeutic quantity in segments 600 and 604. Clear (plain) inner segments 602 and 606 contain pharmacologically ineffective amounts of each of the three drugs found in the tablet. Interfaces 610, 612, 614, and 616 represent the regions at which two contiguous segments adjoin. The tablet of Fig. 5 is provided with a different color for each segment. Even though there is no surface scoring or indicia, the color scheme is such that a person's attention may be directed to apply force to break the tablet through segment 602 to create the tablettes depicted in Figs. 6a and 6b. Fig. 6a depicts the smaller tablette created by breaking the tablet of Fig. 5 through segment 602 in a transverse fashion. Segment 620 has been created by said breaking, and segment 602 of Fig. 5 no longer exists as a intact segment. Fig. 6b depicts the larger tablette created by said breaking of the tablet of Fig. 5. New upper segment 622 has been created.

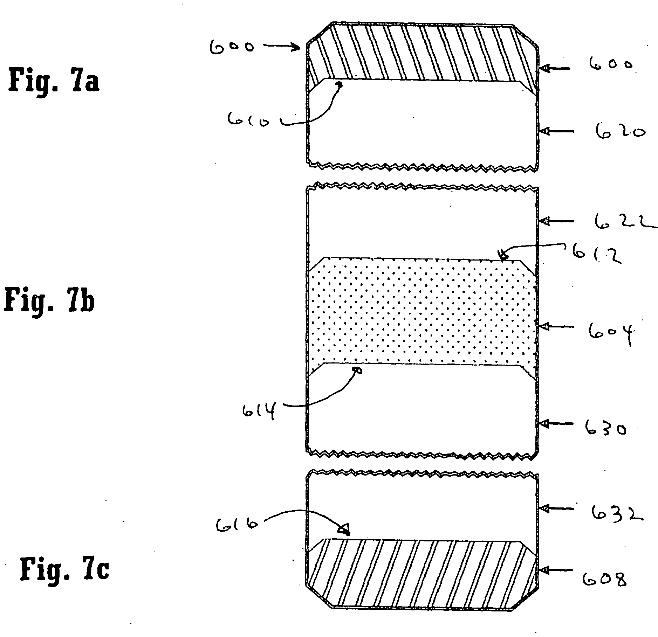

Figs. 7a-c depict three tablettes created by the subsequent breaking of the tablette of Fig. 6b. New segment 630 and segment 632 have been created and segment 606 no longer exists as an intact segment.

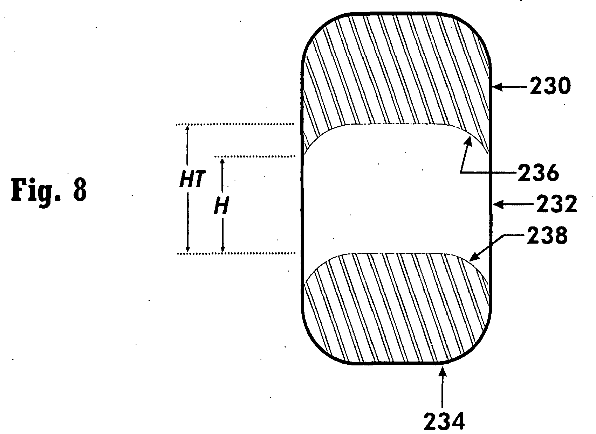

Fig. 8 is a cross-section of a three segment tablet, having a top segment 230 having a drug, an inactive middle segment (no detectable drug or a pharmacologically ineffective amount of a drug) and a bottom segment which contains the same drug as the top segment. This tablet is formed with a tablet punch having a curved profile which forms curved interfaces 236 and 238 as well as the top of the tablet. The effective height of the middle segment is H which is less than the actual height HT of the middle segment due to the effect of the curved tablet punch. It is desired to break this type of a tablet only through that part of the middle segment within the effective height H to avoid breaking into the drug containing top or bottom segment.
The above-described tablet contains three layers and three segments may contain amlodipine. It may be broken through the segment formed from the inactive granulation. Said breaking, if confined solely to said middle granulation, will create two tablettes, each containing one substantially intact segment comprising amlodipine and a part of the middle segment. The advance on the art of tablet splitting is that maximal accuracy of dosing present in each tablette will be achieved, since any weight (or, mass) difference between the two tablettes will be due to differences in the quantity of middle segment present, but said middle segment is expected to have little if any amlodipine therein. Similarly, any loss of mass due to chipping or crumbling is expected to occur in the middle segment.

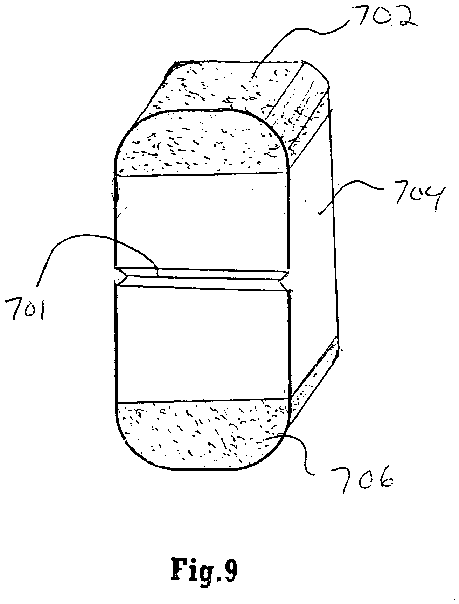

Fig. 9 is a perspective view of a tablet of the invention which shows score 701 as a separating mark on a front surface and top active (drug containing) segment 702; middle inactive segment (no detectable drug or a pharmacologically ineffective amount of a drug) and bottom active segment 706. When the tablet is broken at the score 701, the top segment and the bottom segment will remain intact.

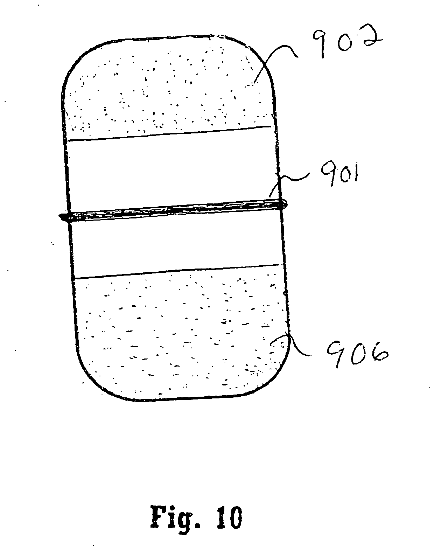

Fig. 10 is a front view of a tablet of the invention showing a band 901, such as a gelatin band that is used to seal hard gelatin capsules, which is applied to suitable tablets according to the invention to provide a separating mark. Techniques such as those use to band capsules, as disclosed in U.S. 4,922,682, which is incorporated by reference, may be modified to provide a band in making tablets according to the invention.

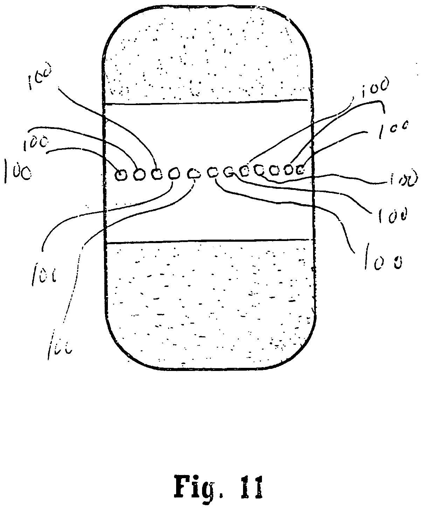

Fig. 11 shows a series of perforations 100 that may be made in the surface of a tablet to form a separation mark according to the invention. These perforations may be formed e.g. by mechanical or laser drilling 1-2mm holes that extend into the surface to a depth of 1-2mm.

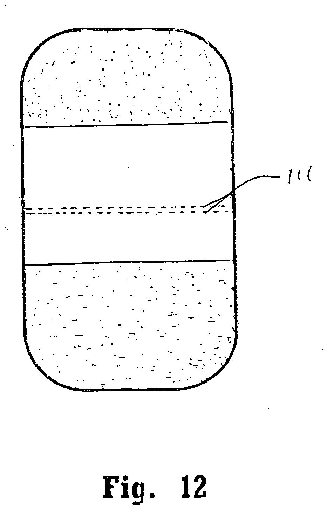

Fig. 12 shows a front view of a tablet according to the invention that has two printed dotted lines that serve as a separation mark according to the invention.

Another preferred embodiment of the invention utilizes a variation on the above, for example:
A first granulation comprising hydrochlorothiazide (HCTZ) enters the die, followed by an inactive granulation entering the die twice, followed at the fourth and final filling station by a granulation comprising bisoprolol (a beta-blocker). After final compression, a tablet consisting of three segments (formed from four layers) has been created. The layer formed from the first granulation is the bottom layer, the layers formed from inactive excipients are the two inner layers and together, after tablet formation, make up the middle (inner) segment, and the final granulation comprises the top layer, which after final compression is denoted the top segment. Thus all dimensions and directions herein relate to the method of manufacture of the tablet. This preferably taller than wider tablet may contain some amount of HCTZ in the middle and top segments, and may contain some amount of bisoprolol in the middle and bottom segments.

After breaking the above tablet entirely through the middle segment, two tablettes are formed. One contains primarily the full, presumably therapeutically effective quantity of HCTZ and probably some amount, preferably trace, of bisoprolol; the other contains primarily the full amount of bisoprolol and probably some amount, preferably trace, of HCTZ, plus some quantity of said middle segment. Important therapeutic benefits in terms of dosage adjustment, side effect management, and the like are obtained from the above tablet design and optional ability to substantially completely create two individual dosage forms form the combination product.

As Fig.8 demonstrates, cupping or beveling of the upper punch commonly causes the peripheral parts of any segment other than the lowest segment, to extend below the level of the central part of that segment. In order to fully realize the benefit of a "separating segment" per the invention, it is optimal that a transverse plane be able to be placed between the lowest part of a superiorly disposed segment, and the highest part of an inferiorly disposed segment, with said plane passing between an interposed, preferably pharmacologically inactive segment. The vertical distance between the lowest part of a superiorly disposed segment and the highest part of an inferiorly disposed segment is herein denoted the effective height H. Generally, that measurement will be from the vertical height from the bottom of the tablet to the plane drawn horizontally from the periphery of the higher segment, due to the cupping or beveling of such a segment, and from the vertical height from the bottom of the tablet to the center of the lower segment.

The effective height in the case of beveling or cupping of segments, as easily reflected in the shape of the top of the tablet, is always less than the height of the separating or interposed segment through which braking is intended to occur. The height of an interposed segment is the vertical distance from its highest point to the highest point of the contiguous superiorly disposed segment.

In the case of separating or interposed segments, prior art limits the height to approximately 1 mm for immediate release pharmaceutical tablets. The effective height H has been limited to less than that.

Preferred tablets of the invention often use a height and an effective height H that are both over 4 mm, and may exceed 6 mm. Lesser heights and effective heights are utilized when needed due to size constraints on the tablet.

### DESCRIPTION OF MANUFACTURE OF PREFERRED EMBODIMENTS

A tablet is made which has three segments, each with an active top or upper segment and an active lower or bottom segment separated by a substantially inactive middle segment. A Stokes 27-station tri-layer rotary tablet press is used. All formulations are directly compressible powder blends. The blending both of the amlodipine formulation and the benazepril formulation are performed in a Patterson-Kelly "V" blender. The middle segment consists of 194 mg of Nu-Tab^{®} and requires no blending. The tablets are compressed using 0.131 inch by 0.3222 inch oval, concave tablet punches to a hardness of 35 kiloponds. The bottom segment is introduced first into the die. The tablet weight is 310 mg. Tablets so made are 8 mm tall; the inactive middle segment varies from 5-6 mm in height and a width of 4mm.

Weights in mg of the granulation comprising each segment are as follow:

| Bottom Segment | Mg. |
|---|---|
| Dibasic calcium phosphate anhydrous | 51.13 |
| Amlodipine besylate | 7.15 |
| Sodium starch glycolate (Explotab^{®}) | 2.48 |
| Magnesium stearate | 0.93 |
| FD&C Blue #1 Aluminum Lake | 0.31 |
| Total | 62.00 |

### Manufacturing Instructions

1. Weigh each ingredient
2. Screen each ingredient
3. Triturate the color with the major diluent in geometric proportions using a suitable mixer
4. Add the remaining ingredients, except the lubricant, to the color mixer from step #3 and mix for desired time
5. Add the lubricant to the blend from Step #4 and mix for desired time
6. Add the blend to a suitable press fitted with the desired tooling and compress into tablets

| Middle Segment | Mg. |
|---|---|
| | |
| Nu-Tab^{®} (Compressible sugar 30/35 N.F.) | 194.00 |
| | |

| Top Segment | Mg. |
|---|---|
| | |
| Lactose 310 monohydrate | 42.03 |
| Benazepril HCl | 9.00 |
| Crospovidone | 2.16 |
| Magnesium stearate | 0.54 |
| FD&C Red #40 Aluminum Lake | 0.27 |
| Total | 54.00 |

### Manufacturing Instructions

1. Weigh each ingredient
2. Screen each ingredient
3. Triturate the color with the major diluent in geometric proportions using a suitable mixer
4. Add the remaining ingredients, except the lubricant, to the color mixer from step #3 and mix for desired time
5. Add the lubricant to the blend from Step #4 and mix for desired time.
6. Add the blend to a suitable press fitted with the desired tooling and compress into tablets

### Tabletting Instructions

1. Place the powder for active layer in hopper #1.
2. Place the powder for placebo layer in hopper #2
3. Place the powder for active layer in hopper #3
4. Compress layer #1 tablets to desired weight (tablets for layer #1 should form a soft compact)
5. Compress layer #1 & Layer #2 tablets to desired combined weight of layer #1 and layer #2 weight (tablets should form a soft compact)
6. Compress the tri-layer tablet to the desired total tablet weight (layer #1 weight + layer #2 weight + layer #3 weight) Tablet should be at desired hardness.

A similar tablet of the invention is separately produced using the same top and bottom segments as the above, but using the following ingredients instead of Nu-Tab for the middle segment. The following are blended using a Patterson-Kelly "V" blender.

| Ingredients for middle segment: | Mg. |
|---|---|
| Dibasic calcium phosphate anhydrous | 158.59 |
| Magnesium stearate | 2.79 |
| PVP K-30 | 2.62 |
| Total | 164.00 |

### Manufacturing Instructions

1. Weigh each ingredient
2. Screen each ingredient
3. Place all of the ingredients, except the lubricant, into a suitable mixer and mix for desired time
4. Add the lubricant to the blend from Step #3 and mix for desired time.
5. Add the blend to a suitable press fitted with the desired tooling and compress into tablets

The tablets were compressed using oval 0.131 inch by 0.3222 inch, concave tablet punches to a hardness of 35 kiloponds. The bottom segment was introduced first into the die. The tablet weight was 280 mg. Tablets with said middle segment were 6 mm high, and the inactive middle segment was 3.5-4 mm high.

### Tabletting Instructions

1. Place the powder for active layer in hopper #1.
2. Place the powder for placebo layer in hopper #2
3. Place the powder for active layer in hopper #3
4. Compress layer #1 tablets to desired weight (tablets for layer #1 should form a soft compact)
5. Compress layer #1 & Layer #2 tablets to desired combined weight of layer #1 and layer #2 weight (tablets should form a soft compact)
6. Compress the tri-layer tablet to the desired total tablet weight (layer #1 weight + layer #2 weight + layer #3 weight) Tablet should be at desired hardness.

In a similar way, other taller than wider tablets can be made on a tablet press, such as, the Korsch TRP900 which can produce taller tablets due to its design for deep filling cams which allow for deeper fills and greater distances between the upper and lower compression tools. To make an oval 0.131 inch by 0.3222 inch, concave tablet that is 12mm tall on the Korsch TRP900 the formulator would have to increase the weight of the inactive Nu-Tab^{®} middle segment to about 323mg. Similarly to have a finished tablet height of 14mm the tablet would be formulated with a middle segment weighing about 388mg. If the formulator preferred, they could use the second example for a middle layer, i.e., the dibasic calcium phosphate (DCP) formulation. In such a case making an oval 0.131 inch by 0.3222 inch, concave tablet that is 12mm tall on the Korsch TRP900 the formulator would have to increase the weight of the inactive DCP middle segment to about 410mg. Similarly to have a finished tablet height of 14mm the tablet would be formulated with a middle segment weighing about 492mg.

One or more drugs via the dosage forms such as tablets and tablettes of the invention may be administered to a patient, mammal, or other animal in need of pharmaceuticals for the prevention or treatment of an illness, maintenance of good health, retarding of aging, or other purpose. A patient may be treated with only one drug from a combination product, such as with a novel tablette of the invention, enabling downward dose adjustment for a variety of reasons; or, in a similar vein, a patient may be treated with one whole tablet containing a plurality of active drugs and in addition receive only one drug from a similar tablet, thus enabling upward dose adjustment. Combination products that can benefit from the invention, in which one drug is in an outer active segment, and a second and different drug is in the other outer active segment, and a pharmacologically ineffective inner segment as in embodiments such as was described above, include those containing the following pairs of drugs: amlodipine and either benazepril, chlorthalidone, or atorvastatin; benazepril and hydrochlorothiazide; olmesartan and hydrochlorothiazide; and many others, including the majority of the currently-produced combination products. Also included is the method of treating a patient with a precise partial dose of medication from a whole tablet, which may be a half or quarter of the whole dose, but may usefully be a different fraction. Warfarin especially may usefully be produced and dosed according to the invention with separable segments of the tablet that may but need not be as halves, quarters, etc. L-thyroxine and digoxin are other examples that could so benefit, along with warfarin.

The following give possible clinical situations in which the tablets of the invention could provide important benefits.
1. A currently marketed product in the United States is Caduet^{®}, which contains the active ingredients atorvastatin calcium (atorvastatin) and amlodipine besylate (amlodipine) which are largely homogeneously interdispersed in an unscored tablet. The product is indicated to treat both hyperlipidemia (atorvastatin) and hypertension (amlodipine). A patient ingesting this tablet daily may then undergo a blood test and be diagnosed as having liver dysfunction as manifested by elevation of an enzyme's concentration in the blood. The physician may then recommend cessation, possibly temporary, of atorvastatin, which is stated by the manufacturer to be a possible cause of liver dysfunction. A patient receiving Caduet, however, would have to thus also discontinue amlodipine, which is not in this example desired by the physician. A tablet of the invention in which atorvastatin and amlodipine were segregated in different outer active segments, separated by a middle segment of adequate dimensions, would be a clear advance over the current Caduet formulation, because such a tablet would allow a patient to promptly continue ingesting amlodipine while stopping ingestion of atorvastatin, without having to go to a pharmacy and fill a new prescription for a tablet containing only amlodipine as the active ingredient, while having previously had the convenience of having both drugs combined in a single dosage form. The above embodiment of the invention represents an improvement over the current Caduet dosage form.
   Another clinical situation in which the invention is superior to Caduet is one in which a patient receiving amlodipine 5 mg once daily and atorvastatin 20 mg once daily is advised by a physician to increase the daily amlodipine dose to 10 mg once daily. A patient in possession of adequate tablets of the invention, with the active drugs segregated in a three-segment tablet, would be able to promptly increase the amlodipine dose by taking a whole tablet of the invention once daily, plus a tablette containing 5 mg of amlodipine, produced by breaking a second whole tablet of the invention.
   Another clinical situation in which the invention is superior to Caduet involves the case in which a physician wishes a patient to ingest atorvastatin 20 mg each morning and amlodipine 2.5 mg twice daily. The invention provides for amlodipine to be separated from atorvastatin and then broken precisely in half. The invention thus allows the patient the advantage of one tablet, whereas to accomplish this currently in the United States would require one 20 mg Lipitor^{®} (atorvastatin) tablet and two Norvasc^{®} (amlodipine) 2.5 mg tablets.
2. The combination of amlodipine besylate and benazepril hydrochloride (benazepril) is marketed in the United States under the brand name of Lotrel^{®}. This product is a capsule that is routinely ingested whole. An embodiment of the invention provides a whole tablet containing one outer segment containing amlodipine as the only active drug and the other outer segment containing benazepril as the only active drug. If desired, either outer layer may be formed into more than one segment, as in Fig. 1a. As in example 1 above regarding Caduet, the middle segment is inactive and may be broken through to create two tablettes, each comprising a whole amount of each outer active segment plus approximately half of the amount of the middle inactive segment. If a patient were to develop a need for double the dose of one active drug but not the other, the tablet of the invention could meet that need. Alternatively, if a patient were to develop a need to ingest only one active drug, possibly temporarily, due to such conditions as blood pressure changes or a side effect to one drug but not the other, the tablet of the invention allows this to be done without a new dosage form being prescribed.
3. Another use of the invention involves the combination of amlodipine and chlorthalidone or another diuretic, which may usefully be combined to treat hypertension. Benefits of the invention are similar to those described in the paragraph immediately preceding this paragraph.
4. Another use of the invention involves the combination of olmesartan medoxomil (olmesartan, an angiotensin receptor blocker) and hydrochlorothiazide (HCTZ). This product is currently marketed in the United States under the name Benicar/HCT^{®}, with the doses, respectively, of, in mg: 20/12.5, 40/12.5, and 40/25. A very common starting dose of a patient will be 20/12.5 once daily. The product is currently marketed in all strengths as a homogeneous tablet containing both active drugs. Formulated according to the current invention, a patient who begins treatment with the 20/12.5 dose may be increased with the same tablet to each of the other doses by ingesting one whole 20/12.5 tablet and either a half tablet containing 20 mg of olmesartan or a half tablet containing 25 mg of HCTZ. This will provide the physician an opportunity to investigate the new dose before giving the patient a new prescription. Other advantages of the invention are similar to those described above.
5. Another useful combination product that may be formulated according to the invention involves angiotensin converting enzyme inhibitors (ACEs) and diuretics such as HCTZ. Both types of drug not uncommonly have side effects, so that the invention will be useful to physicians in dealing with the side effects, as well as with changing dosing needs to deal with the anti-hypertensive and other clinical benefits of the drugs.
6. Another product that may benefit from the invention regarding separating active drugs in separate outer layers with an inactive middle segment (layer) is a combination product containing two active drugs, fluoxetine and olanzapine.

No limitation to the above therapeutic fields or to the specific examples within their fields is intended for tablets of the invention, which may be used in any suitable combination of drugs. No limitation to two-drug combinations exists, as well. For instance, one outer active segment of a tablet according to the invention could contain levodopa and carbidopa, and the other outer active segment could contain entacapone, a tablet product containing all three drugs in a homogeneous fashion that is currently marketed in the United States as Stalevo^{®}. Also, a tablet per the invention could involve five layered segments, with, for example, amlodipine in one outer segment, an inactive segment adjoining it, a middle segment containing chlorthalidone or HCTZ, and a second inactive Segment adjoining both it and the other outer segment that contains benazepril (see Fig. 8). If both inactive segments were of adequate dimensions to be conveniently breakable without damaging any of the three active segments, thus providing significant clinical advantages due to the adoption of flexible dosing of the different active segments.

The following list of possible combinations of a plurality of drugs is exemplary and not limiting. The combinations referred to may include two or more members of the classes listed. Drugs listed below, and herein, may for convenience exclude mention of any salt of a drug; e.g., "atorvastatin" is listed even though its marketed form is atorvastatin calcium.

Without limitation, useful combinations may include a plurality of drugs from within the following six drug classes.

In addition, tablets of the invention may be created containing only one of a drug from the following list. With regards to combination use, two methods of use may apply to the invention. One of these methods is to place an individual drug in a granulation and a different individual drug (or combination of drugs) in a different granulation, potentially with an inactive granulation interposed between them; another method is to place a plurality of drugs in one or more segments.
1. Anti-anginal agents, for example:
   A. Calcium antagonists (see list below);
   B. Beta-blocker (see list below);
   C. Organic nitrate preparation (e.g., isosorbide mononitrate or dinitrate).
2. Anti-anginal agent plus an anti-platelet agent, such as aspirin, clopidogrel, or ticlopidine.
3. Two hypoglycemic agents (see list below).
4. Potassium chloride and any thiazide-type or loop diuretic (see lists below).
5. Lipid-lowering agent plus: hypoglycemic agent, anti-platelet agent, anti-anginal agent, and/or antihypertensive agent (see lists above and below)
   Hypoglycemic agents include: thiazolidinediones: pioglitazone, rosiglitazone; sulfonylureas: glyburide, glipizide, glimepiride, chlorpropamide;
   Biguanides: metformin;
   Meglitinides: nateglinide, repaglinide;
   Glucosidase inhibitors: acarbose, miglitol.
6. Antihypertensive agents:
   Beta-blockers: acebutolol, atenolol, bisoprolol, celiprolol, metoprolol, mebivolol, carvedilol (a mixed alpha-beta blocker), nadolol, oxprenolol, penbutolol, pindolol, propranolol, timolol, betaxolol, carteolol;
   Calcium antagonists (calcium-channel blockers): nifedipine, amlodipine, verapamil, diltiazem, nisoldipine, felodipine, isradipine, lacidipine, lercanidipine, nicardipine, manidipine;
   Thiazide-type diuretics (with or without potassium-retaining diuretics such as triamterene, amiloride, or spironolactone): hydrochlorothiazide, chlorothiazide, cyclopenthiazide, polythiazide, bendrofluazide, hydroflumethiazide, chlorthalidone, indapamide, methylclothiazide, metolazone;
   Angiotensin converting enzyme inhibitors: captopril, enalapril, lisinopril, ramipril, trandolapril, quinapril, perindopril, moexipril, benazepril, fosinopril;
   Angiotensin receptor blockers: losartan, valsartan, candesartan, telmisartan, eprosartan, irbesartan;
   High-ceiling (loop) diuretics (with or without potassium-retaining diuretics such as triamterene, amiloride, or spironolactone): furosemide, torsemide, ethacrynic acid, bumetamide;
   Aldosterone antagonist diuretics: spironolactone, eplerenone;
   Alpha-blockers: doxazosin, terazosin, prazosin, indoramin, labetolol (a mixed alpha-beta blocker);
   Central alpha-agonists: clonidine, methyldopa;
   Imidazoline: moxonidine;
   Direct vasodilators: hydralazine, minoxidil;
   Adrenergic neuronal blocker: guanethidine.

Lipid-lowering agents include:
Statins: lovastatin, simvastatin, pravastatin, rosuvastatin, atorvastatin, fluvastatin;
Fibrates: clofibrate, bezafibrate, fenofibrate, gemfibrozil, ciprofibrate;
Others: ezetimide, niacin, acipimox.

The combinations of drugs disclosed herein are for illustrative purposes and are not intended to limit the scope of the invention.

Regarding the important usage of the tablets and tablettes of the invention, that involving division of a tablet into tablettes containing similar active segments, most drugs that may undergo dosage adjustment will be preferred if they may be divided in an optimally precise manner. Examples of drugs that will especially benefit from the advances of the invention in this manner include narrow therapeutic index drugs such as warfarin, digoxin, L-thyroxine; vasoactive drugs such as amlodipine; hypoglycemic agents such as rosiglitazone and glipizide; and anxiolytics drugs such as alprazolam. These are however but a small fraction of the great mass of drugs that will benefit from the various embodiments and procedures of the invention.

There are numerous methods of use of the dosage forms of the invention, including its tablets and tablettes. Persons skilled in the medical and pharmaceutical arts will recognize the many advantages that the various embodiments of the invention allow over current products. Some examples of benefits of the inventions involving tablets containing exactly one similar active segment are described immediately below.
1. Warfarin is an anticoagulant marketed in the U.S. under the brand name Coumadin^{®}, which is a scored tablet. Research has shown that patients do not break warfarin 5 mg tablets into equal 2.5 mg segments. The invention teaches different types of tablets that allow warfarin tablets of any common human dose to be broken into precise halves, and potentially precise thirds, quarters, etc. Thus a patient may utilize warfarin half-tablets produced as per the invention with similar confidence as in the whole tablet. Because warfarin doses are frequently broken, many clinical scenarios exist in which the invention will benefit patients.
2. Norvasc (amlodipine besylate or amlodipine herein) is marketed as unscored 2.5, 5, and 10 mg tablets in the U.S. These tablets are of irregular shape and are difficult to break. The FDA-approved dosage range is from 2.5 to 10 mg ingested orally daily. The invention allows improved functionality of amlodipine. For example, under the invention, a patient receiving 5 mg daily who a physician wishes to increase to 7.5 mg daily may simply utilize a tablet of the invention that comprises two separate 2.5 mg segments to increase the dose to precisely 7.5 mg, such as by ingesting one whole 5 mg tablet and one 2.5 mg tablette created by breaking a 5 mg tablet into two tablettes each containing 2.5 mg of amlodipine. Convenience and cost savings are clear. Similarly, a patient receiving a 10 mg dose of Norvasc who is advised to reduce the dose to 5 mg daily must currently purchase a new prescription for 5 mg Norvasc tablets. The invention provides the ability to provide a 10 mg tablet that may be broken into two tablettes, each containing precisely 5 mg of amlodipine. The invention may therefore enable greater flexibility of treating patients, and provide cost savings as well. A further benefit of the invention is that various embodiments allow fully accurate separation of a tablet into a tablette comprising one-fourth of the dose of the active ingredient as is found in the whole tablet. This may for example be done for amlodipine by providing four active segments all containing 2.5 mg of amlodipine and all contiguous with the same side of an inactive outer segment (see embodiment #1; and see Fig. 6a modified to have four and not two active segments). Thus, a 10 mg amlodipine tablet of the invention may be utilized to provide a 7.5 mg dose; or, it may be utilized to provide four 2.5 mg doses.

A further benefit of the invention may relate to pediatric or geriatric doses, which may not be produced in appropriate dose strengths. In the case of amlodipine, a 1.25 mg daily dose may be useful in either small children with hypertension, or in frail elderly patients with angina or hypertension, who may have hepatic dysfunction. Even though the United States Food and Drug Administration (FDA) has not approved a 1.25 mg dose, precise divisibility of the approved 2.5 mg dose would allow a 1.25 mg daily dose. In addition, precise divisibility of the approved 2.5 mg dose will allow accurate dosing of 3.75 mg daily.

Another use of the invention is to for the first time enable a method of cost savings to insurers and patients. The invention allows this because many drugs, such as Norvasc and Coumadin, have pricing that differs little (if at all) between different doses. Because tablet splitting is imprecise for most scored tablets, the practice of mandatory splitting has been met with disapproval by most physician and pharmacist organizations. The invention enables tablet splitting due to provide accurate dosing when a tablet (or some tablettes, as in Fig. 1b) of the invention are broken as described herein. Substantial benefits are foreseen from this innovation. In addition, the ability to separate one active drug from another in a combination product has cost saving advantages, as well.

## Claims

1. An immediate release pharmaceutical tablet obtainable by production on a layer press and comprising vertically disposed segments wherein the drug-containing segments are separated by an inner segment that lacks a pharmacologically effective quantity of drug and the height of which is great enough to allow said inner segment to serve as breaking region, said tablet containing a drug or drugs, in which:
(a) said tablet includes at least one segment created from an inactive granulation and all segments that contain a pharmacologically effective dose of a drug or drug contain the same drug or combination of drugs at the same ratio;
or,
(b) said tablet includes: (i) a first segment containing a drug or drugs; (ii) a third segment containing a drug or plurality of drugs which is/ are different from the drug or drugs in said first segment, and (iii) a second segment that is interposed between said first and third segments and that has an undetectable amount of, or else a pharmacologically ineffective amount of, any drug present in said tablet; (iv) physical and chemical compatibility between said first and third segment;
or,
(c) said tablet includes: (i) a first segment containing a drug or two or more drugs; (ii) a third segment containing a drugs or drugs which are different from the drug or drugs in said first segment; (iii) a second segment that is interposed between said first and said third segment and that has an undetectable amount of, or a pharmacologically ineffective amount of, any drug present in said tablet; and, (iv) said third segment has either or both of the following: a height of at least 2.5 mm or an effective height of at least 1.5 mm.

2. An immediate release pharmaceutical tablet as defined in claim 1 comprising at least two compositionally distinct segments, with a first segment containing a drug in a pharmacologically effective amount and a second segment that:
(b) has no detectable drug or the same drug which is in said first segment in a pharmacologically ineffective amount and also includes a third segment having the same drug that is present in said first segment; or
(d) either has no detectable drug, or lacks any drug or drugs in a pharmacologically effective amount, wherein said tablet also comprises a third segment containing a pharmacologically effective quantity of a different drug from the drug that is present in said first segment and wherein said first and third segments are physically and chemically compatible;
or,
(e) either contains no detectable drug or else contains in a pharmacologically ineffective amount the same drug which is in said first segment; and also includes a third segment having a pharmacologically effective dose of a different drug or drugs from said drug or drugs that is/are present in said first segment, said second segment having one or both of the following: a height of at least 2.5 mm or an effective height of 1.5 mm.

3. An immediate release pharmaceutical tablet as defined in claim 2 wherein said second segment has a height greater than the combined height of said first segment and said third segment.

4. An immediate release pharmaceutical tablet as defined in claim 2 where a score, perforations, printed marks, or gelatin or a combination of thereof, are placed on or within said second segment and are predominantly horizontally oriented to guide tablet breaking through said second segment substantially without breaking through said first segment or said third segment.

5. An immediate release pharmaceutical tablet as defined in claim 2 wherein the second segment contains a combination of the drug in said first segment with another drug or drugs not present in said first segment.

6. An immediate release pharmaceutical tablet as defined in claim 2 wherein the second segment contains no detectable drug or the same drug which is in said first segment in a pharmacologically ineffective amount, and wherein said tablet has a third segment having the same drug that is present in said first segment.

7. An immediate release pharmaceutical tablet as defined in claim 6 wherein the concentration of the drug in said first and third segments is substantially the same.

8. An immediate release pharmaceutical tablet as defined in claim 7 in which the quantity of drug in said first and third segments is substantially the same.

9. An immediate release pharmaceutical tablet as defined in claim 7 wherein said first and second segments come together at an interface which is provided with a separation mark.

10. An immediate release pharmaceutical tablet as defined in claim 2 having in said second segment no detectable drug or the same drug which is in said first segment in a pharmacologically ineffective amount, said tablet also containing a third segment having a different drug from the drug that is present in said first segment, said third segment being physically and chemically compatible with said first segment.

11. An immediate release pharmaceutical tablet as defined in claim 2 that contains a separation mark selected from the group consisting of a score, indicia, printed indicia, a perforation or a band in a second segment or at an interface between segments.

12. An immediate release pharmaceutical tablet as defined in claim 6 that is derived from three vertically disposed granulations that form three vertically disposed segments, a first segment at the top, a second segment in the middle and a third segment at the bottom and having a substantially horizontal separation mark placed substantially horizontally on or within said second segment.

13. An immediate release pharmaceutical tablet as defined in claim 6 in which said first segment and said third segment are substantially compositionally identical.

14. An immediate release pharmaceutical tablet as defined in claim 6 in which said first segment and said third segment contain substantially identical quantities of the same drug or drugs, where the amount or ratio of said drug or drugs is the same in both segments.

15. An immediate release pharmaceutical tablet as defined in claim 6 in which said second segment is of a color which permits visual identification of said second segment apart from said first segment.

16. An immediate release pharmaceutical tablet as defined in claim 6 in which said second segment contains a separation mark.

17. An immediate release pharmaceutical tablet as defined in claim 16 in which said second segment contains indicia.

18. An immediate release pharmaceutical tablet as defined in claim 17 in which said second segment contains printed indicia.

19. An immediate release pharmaceutical tablet as defined in any one of claims 16, 17, or 18 in which breaking said tablet, when guided by color, scoring, or printed indicia yields a tablette having a predetermined quantity of drug.

20. An immediate release pharmaceutical tablet as defined in claim 6 in which the first segment and third segment consist essentially of the same drug.

21. An immediate release pharmaceutical tablet as defined in claim 10 is provided with a separation mark.

22. An immediate release pharmaceutical tablet as defined in claims 6 or 10 wherein the second segment has a color which permits visual identification of said second segment apart from said first segment and said third segment.

23. An immediate release pharmaceutical tablet as defined in claims 6 or 10 in which the second segment corresponds to a segment located in the middle of the tablet.

24. An immediate release pharmaceutical tablet as defined in claim 1 wherein said core tablet forms a tablet structure selected from the group consisting of A-1-B-I-A, A-I-B-I-B, A-I-B-I-C, or A-B-I-C wherein A, B, C and I represent mutually different segments that are vertically disposed upon each other and wherein A, B and C contain an active drug or drugs and I is a segment without a pharmacologically effective quantity of any drug.

25. An immediate release pharmaceutical tablet as defined in claim 24 in which each of segments A, B, C and/or I have either a color or indicia which identifies each of segments A, B, I, and C as being different segments.

26. A method of breaking an immediate release pharmaceutical tablet as defined in claims 11, or 16 which comprises breaking said tablet at said separation mark.

27. A pharmaceutical tablet as defined in claim 1 in which said drug or drugs is or are pharmacologically effective in the treatment of cardiovascular conditions, psychiatric conditions, diabetes, thyroid disorders, pain or thrombotic disorders.

28. A partial dose of a drug contained in a pharmaceutical tablet as defined in claims 11, or 16, derived from the break of said pharmaceutical tablet at said separation mark to form two or more tablettes for the administration of at least one of said tablettes to a patient.

## Patentansprüche

1. Pharmazeutische Tablette mit sofortiger Freisetzung, die durch Herstellung in einer Tablettenpresse erhalten werden kann und vertikal angeordnete Segmente umfasst, wobei die wirkstoffhaltigen Segmente durch ein inneres Segment getrennt sind, das keine pharmakologisch wirksame Wirkstoffmenge enthält und dessen Höhe groß genug ist, um zu gestatten, dass dieses innere Segment als Bruchbereich dient, wobei diese Tablette einen Wirkstoff oder Wirkstoffe enthält, wobei:
(a) diese Tablette mindestens ein aus einer inaktiven Granulation erzeugtes Segment umfasst und alle Segmente, die eine pharmakologisch wirksame Dosis eines Wirkstoffs oder von Wirkstoffen enthalten, den gleichen Wirkstoff oder die gleiche Kombination von Wirkstoffen im gleichen Verhältnis enthalten;
oder,
(b) diese Tablette Folgendes umfasst: (i) ein erstes Segment, das einen Wirkstoff oder Wirkstoffe enthält; (ii) ein drittes Segment, das einen Wirkstoff oder eine Vielzahl von Wirkstoffen enthält, der/die verschieden von dem Wirkstoff oder den Wirkstoffen im ersten Segment ist/sind; und (iii) ein zweites Segment, das zwischen das erste und das dritte Segment eingefügt ist und eine nicht nachweisbare Menge oder andernfalls eine pharmakologisch unwirksame Menge eines beliebigen in dieser Tablette vorhandenen Wirkstoffs aufweist; (iv) physikalische und chemische Kompatibilität zwischen dem ersten und dem dritten Segment;
oder,
(c) diese Tablette Folgendes umfasst: (i) ein erstes Segment, das einen Wirkstoff oder zwei oder mehr Wirkstoffe enthält; (ii) ein drittes Segment, das einen Wirkstoff oder Wirkstoffe enthält, der/die verschieden von dem Wirkstoff oder den Wirkstoffen im ersten Segment ist/sind; (iii) ein zweites Segment, das zwischen das erste und das dritte Segment eingefügt ist und das eine nicht nachweisbare Menge oder eine pharmakologisch unwirksame Menge eines beliebigen in dieser Tablette vorhandenen Wirkstoffs aufweist; und wobei (iv) das dritte Segment eines oder beides von Folgendem aufweist: eine Höhe von mindestens 2,5 mm oder eine effektive Höhe von mindestens 1,5 mm.

2. Pharmazeutische Tablette mit sofortiger Freisetzung nach Anspruch 1, die mindestens zwei Segmente mit unterschiedlicher Zusammensetzung umfasst, wobei sie ein erstes Segment, das einen Wirkstoff in einer pharmakologisch wirksamen Menge enthält, und ein zweites Segment aufweist, das:
(b) keinen nachweisbaren Wirkstoff oder den gleichen Wirkstoff, der im ersten Segment enthalten ist, in einer pharmakologisch unwirksamen Menge aufweist und außerdem ein drittes Segment umfasst, das den gleichen Wirkstoff aufweist, der im ersten Segment vorhanden ist; oder
(d) entweder keinen nachweisbaren Wirkstoff aufweist oder keinerlei Wirkstoff oder Wirkstoffe in einer pharmakologisch wirksamen Menge aufweist, wobei diese Tablette ferner ein drittes Segment umfasst, das eine pharmakologisch wirksame Menge eines von dem im ersten Segment enthaltenen Wirkstoff verschiedenen Wirkstoffes enthält, und wobei das erste und das dritte Segment physikalisch und chemisch kompatibel sind;
oder,
(e) entweder keinen nachweisbaren Wirkstoff enthält oder andernfalls in einer pharmakologisch unwirksamen Menge den gleichen Wirkstoff enthält, der im ersten Segment enthalten ist; und ferner ein drittes Segment umfasst, das eine pharmakologisch wirksame Dosis eines Wirkstoffs oder von Wirkstoffen aufweist, der/die von dem Wirkstoff oder den Wirkstoffen, der/die im ersten Segment enthalten ist/sind, verschieden ist/sind, wobei das zweite Segment eines oder beides von Folgendem aufweist: eine Höhe von mindestens 2,5 mm oder eine effektive Höhe von 1,5 mm.

3. Pharmazeutische Tablette mit sofortiger Freisetzung nach Anspruch 2, bei der das zweite Segment eine Höhe hat, die größer als die kombinierte Höhe des ersten und des dritten Segments ist.

4. Pharmazeutische Tablette mit sofortiger Freisetzung nach Anspruch 2, bei der eine Kerbe, Perforationen, gedruckte Markierungen oder Gelatine oder eine Kombination davon auf oder im zweiten Segment angeordnet und vornehmlich waagerecht ausgerichtet sind, um das Brechen der Tablette durch dieses zweite Segment im Wesentlichen ohne Brechen durch das erste Segment oder das dritte Segment zu steuern.

5. Pharmazeutische Tablette mit sofortiger Freisetzung nach Anspruch 2, bei der das zweite Segment eine Kombination des Wirkstoffs im ersten Segment mit einem anderen Wirkstoff oder anderen Wirkstoffen enthält, der/die im ersten Segment nicht vorhanden ist/sind.

6. Pharmazeutische Tablette mit sofortiger Freisetzung nach Anspruch 2, bei der das zweite Segment keinen nachweisbaren Wirkstoff oder den gleichen Wirkstoff, der im ersten Segment enthalten ist, in einer pharmakologisch unwirksamen Menge enthält, und wobei diese Tablette ein drittes Segment aufweist, das den gleichen Wirkstoff enthält, der im ersten Segment vorhanden ist.

7. Pharmazeutische Tablette mit sofortiger Freisetzung nach Anspruch 6, bei der die Konzentration des Wirkstoffs im ersten und im dritten Segment im Wesentlichen gleich ist.

8. Pharmazeutische Tablette mit sofortiger Freisetzung nach Anspruch 7, bei der die Wirkstoffmenge im ersten und im dritten Segment im Wesentlichen gleich ist.

9. Pharmazeutische Tablette mit sofortiger Freisetzung nach Anspruch 7, bei der das erste und das zweite Segment an einer Übergangsstelle aufeinander treffen, die mit einer Trennmarkierung versehen ist.

10. Pharmazeutische Tablette mit sofortiger Freisetzung nach Anspruch 2, die im zweiten Segment keinen nachweisbaren Wirkstoff oder den gleichen Wirkstoff, der im ersten Segment enthalten ist, in einer pharmakologisch unwirksamen Menge aufweist, wobei diese Tablette außerdem ein drittes Segment enthält, das einen von dem Wirkstoff, der im ersten Segment vorhanden ist, verschiedenen Wirkstoff aufweist, wobei das dritte Segment physikalisch und chemisch mit dem ersten Segment kompatibel ist.

11. Pharmazeutische Tablette mit sofortiger Freisetzung nach Anspruch 2, die eine Trennmarkierung enthält, die aus der Gruppe ausgewählt ist, die aus einer Kerbe, Zeichen, gedruckten Zeichen, einer Perforation oder einem Band in einem zweiten Segment oder an einer Übergangsstelle zwischen Segmenten besteht.

12. Pharmazeutische Tablette mit sofortiger Freisetzung nach Anspruch 6, die aus drei vertikal angeordneten Granulationen erhalten ist, die drei vertikal angeordnete Segmente, ein erstes Segment oben, ein zweites Segment in der Mitte und ein drittes Segment unten, bilden und eine im Wesentlichen horizontale Trennmarkierung aufweisen, die im Wesentlichen horizontal auf oder im zweiten Segment angeordnet ist.

13. Pharmazeutische Tablette mit sofortiger Freisetzung nach Anspruch 6, bei der das erste Segment und das dritte Segment im Wesentlichen die gleiche Zusammensetzung haben.

14. Pharmazeutische Tablette mit sofortiger Freisetzung nach Anspruch 6, bei der das erste Segment und das dritte Segment im Wesentlichen gleiche Mengen des gleichen Wirkstoffs oder der gleichen Wirkstoffe enthalten, wobei die Menge oder das Verhältnis dieses Wirkstoffs oder dieser Wirkstoffe in beiden Segmenten gleich ist.

15. Pharmazeutische Tablette mit sofortiger Freisetzung nach Anspruch 6, bei der das zweite Segment eine Farbe hat, welche die vom ersten Segment gesonderte visuelle Kennzeichnung dieses zweiten Segments gestattet.

16. Pharmazeutische Tablette mit sofortiger Freisetzung nach Anspruch 6, bei der das zweite Segment eine Trennmarkierung enthält.

17. Pharmazeutische Tablette mit sofortiger Freisetzung nach Anspruch 16, bei der das zweite Segment Zeichen enthält.

18. Pharmazeutische Tablette mit sofortiger Freisetzung nach Anspruch 17, bei der das zweite Segment gedruckte Zeichen enthält.

19. Pharmazeutische Tablette mit sofortiger Freisetzung nach einem der Ansprüche 16, 17 oder 18, wobei das Brechen dieser Tablette, wenn es anhand einer Farbe, einer Kerbmarkierung oder gedruckter Zeichen geschieht, eine Tablette mit einer vorbestimmten Wirkstoffmenge ergibt.

20. Pharmazeutische Tablette mit sofortiger Freisetzung nach Anspruch 6, bei der das erste Segment und das dritte Segment im Wesentlichen aus dem gleichen Wirkstoff bestehen.

21. Pharmazeutische Tablette mit sofortiger Freisetzung nach Anspruch 10, die mit einer Trennmarkierung versehen ist.

22. Pharmazeutische Tablette mit sofortiger Freisetzung nach Anspruch 6 oder 10, bei der das zweite Segment eine Farbe hat, welche die vom ersten Segment und vom dritten Segment gesonderte visuelle Kennzeichnung dieses zweiten Segments gestattet.

23. Pharmazeutische Tablette mit sofortiger Freisetzung nach Anspruch 6 oder 10, bei der das zweite Segment einem in der Mitte der Tablette befindlichen Segment entspricht.

24. Pharmazeutische Tablette mit sofortiger Freisetzung nach Anspruch 1, wobei die Kerntablette einen Tablettenaufbau bildet, der aus der aus A-I-B-I-A, A-I-B-I-B, A-I-B-I-C oder A-B-I-C bestehenden Gruppe ausgewählt ist, wobei A, B, C und I voneinander verschiedene Segmente repräsentieren, die vertikal übereinander angeordnet sind und wobei A, B und C einen aktiven Wirkstoff oder Wirkstoffe enthalten und I ein Segment ohne eine pharmakologisch wirksame Menge irgendeines Wirkstoffs ist.

25. Pharmazeutische Tablette mit sofortiger Freisetzung nach Anspruch 24, bei der jedes der Segmente A, B, C und/oder I entweder eine Farbe oder Zeichen aufweist, die jedes der Segmente A, B, I und C als ein verschiedenes Segment kennzeichnet/kennzeichnen.

26. Verfahren zum Brechen einer pharmazeutischen Tablette mit sofortiger Freisetzung nach Anspruch 11 oder 16, welches das Brechen dieser Tablette an der Trennmarkierung umfasst.

27. Pharmazeutische Tablette nach Anspruch 1, bei welcher der Wirkstoff oder die Wirkstoffe in der Behandlung von kardiovaskulären Leiden, psychiatrischen Leiden, Diabetes, Schilddrüsenerkrankungen, Schmerzen oder thrombotischen Erkrankungen pharmakologisch wirksam ist/sind.

28. Teildosis eines in einer pharmazeutischen Tablette nach Anspruch 11 oder 16 enthaltenen Wirkstoffs, die durch das Brechen dieser pharmazeutischen Tablette an der Trennmarkierung erhalten wird, um zwei oder mehr Tabletten für die Verabreichung von mindestens einer dieser Tabletten an einen Patienten zu bilden.

## Revendications

1. Comprimé pharmaceutique à libération immédiate pouvant être obtenu par production sur une presse à couches et comprenant des segments disposés verticalement où les segments contenant un médicament sont séparés par un segment interne qui manque d'une quantité pharmacologiquement efficace de médicament et dont la hauteur est assez grande pour permettre audit segment interne de servir de région de rupture, ledit comprimé contenant un médicament ou des médicaments, dans lesquels :
(a) ledit comprimé inclut au moins un segment créé à partir d'une granulation inactive et tous les segments qui contiennent une dose pharmacologiquement efficace d'un médicament ou un médicament contiennent le même médicament ou une combinaison de médicaments dans la même proportion ;
ou,
(b) ledit comprimé inclut : (i) un premier segment contenant un médicament ou des médicaments ; (ii) un troisième segment contenant un médicament ou une pluralité de médicaments qui est / sont différent(s) du médicament ou des médicaments dans ledit premier segment, et (iii) un deuxième segment qui est intercalé entre lesdits premier et troisième segments et qui a une quantité indétectable, ou bien une quantité pharmacologiquement inefficace, d'un quelconque médicament présent dans ledit comprimé ; (iv) une compatibilité physique et chimique entre ledit premier et troisième segment ;
ou,
(c) ledit comprimé inclut : (i) un premier segment contenant un médicament ou deux ou plusieurs médicaments ; (ii) un troisième segment contenant un médicament ou des médicaments qui sont différents du médicament ou des médicaments dans ledit premier segment ; (iii) un deuxième segment qui est intercalé entre ledit premier et ledit troisième segment et qui a une quantité indétectable, ou une quantité pharmacologiquement inefficace, d'un quelconque médicament présent dans ledit comprimé ; et, (iv) ledit troisième segment a l'une ou l'autre des caractéristiques suivantes, ou les deux : une hauteur d'au moins 2,5 mm ou une hauteur effective d'au moins 1,5 mm.

2. Comprimé pharmaceutique à libération immédiate tel que défini dans la revendication 1 comprenant au moins deux segments distincts en termes de composition, avec un premier segment contenant un médicament en quantité pharmacologiquement efficace et un deuxième segment qui :
(b) n'a aucun médicament détectable ou a le même médicament qui est, dans ledit premier segment, en quantité pharmacologiquement inefficace et inclut également un troisième segment ayant le même médicament qui est présent dans ledit premier segment ; ou
(d) soit n'a aucun médicament détectable, soit manque d'un quelconque médicament ou de médicaments en quantité pharmacologiquement efficace, où ledit comprimé comprend également un troisième segment contenant une quantité pharmacologiquement efficace d'un médicament différent du médicament qui est présent dans ledit premier segment et où lesdits premier et troisième segments sont physiquement et chimiquement compatibles ;
ou,
(e) soit ne contient aucun médicament détectable, soit contient en quantité pharmacologiquement inefficace le même médicament qui est dans ledit premier segment ; et inclut également un troisième segment ayant une dose pharmacologiquement efficace d'un médicament ou de médicaments différent(s) dudit médicament ou desdits médicaments qui est / sont présent(s) dans ledit premier segment, ledit deuxième segment ayant l'une des caractéristiques suivantes, ou les deux : une hauteur d'au moins 2,5 mm ou une hauteur effective de 1,5 mm.

3. Comprimé pharmaceutique à libération immédiate tel que défini dans la revendication 2 où ledit deuxième segment a une hauteur supérieure à la hauteur combinée dudit premier segment et dudit troisième segment.

4. Comprimé pharmaceutique à libération immédiate tel que défini dans la revendication 2 où une marque, des perforations, des marques imprimées, ou de la gélatine ou une combinaison de celles-ci, sont placées sur ou dans ledit deuxième segment et sont principalement orientées horizontalement pour guider le comprimé en perçant ledit deuxième segment substantiellement sans percer ledit premier segment ou ledit troisième segment.

5. Comprimé pharmaceutique à libération immédiate tel que défini dans la revendication 2 où le deuxième segment contient une combinaison du médicament dans ledit premier segment avec un autre médicament ou des médicaments non présent(s) dans ledit premier segment.

6. Comprimé pharmaceutique à libération immédiate tel que défini dans la revendication 2 où le deuxième segment ne contient aucun médicament détectable ou contient le même médicament qui est, dans ledit premier segment, en quantité pharmacologiquement inefficace, et où ledit comprimé a un troisième segment ayant le même médicament qui est présent dans ledit premier segment.

7. Comprimé pharmaceutique à libération immédiate tel que défini dans la revendication 6 où la concentration du médicament dans lesdits premier et troisième segments est substantiellement la même.

8. Comprimé pharmaceutique à libération immédiate tel que défini dans la revendication 7 dans lequel la quantité de médicament dans lesdits premier et troisième segments est substantiellement la même.

9. Comprimé pharmaceutique à libération immédiate tel que défini dans la revendication 7 où lesdits premier et deuxième segments se rejoignent à une interface qui est pourvue d'une marque de séparation.

10. Comprimé pharmaceutique à libération immédiate tel que défini dans la revendication 2 n'ayant, dans ledit deuxième segment, aucun médicament détectable ou ayant le même médicament qui est, dans ledit premier segment, en quantité pharmacologiquement inefficace, ledit comprimé contenant également un troisième segment ayant un médicament différent du médicament qui est présent dans ledit premier segment, ledit troisième segment étant physiquement et chimiquement compatible avec ledit premier segment.

11. Comprimé pharmaceutique à libération immédiate tel que défini dans la revendication 2 qui contient une marque de séparation sélectionnée à partir du groupe consistant en une marque, des repères, des repères imprimés, une perforation ou une bande dans un deuxième segment ou à une interface entre segments.

12. Comprimé pharmaceutique à libération immédiate tel que défini dans la revendication 6 qui est dérivé de trois granulations disposées verticalement qui forment trois segments disposés verticalement, un premier segment au sommet, un deuxième segment au milieu et un troisième segment au fond, et ayant une marque de séparation substantiellement horizontale placée substantiellement horizontalement sur ou dans ledit deuxième segment.

13. Comprimé pharmaceutique à libération immédiate tel que défini dans la revendication 6 dans lequel ledit premier segment et ledit troisième segment sont substantiellement identiques en termes de composition.

14. Comprimé pharmaceutique à libération immédiate tel que défini dans la revendication 6 dans lequel ledit premier segment et ledit troisième segment contiennent substantiellement des quantités identiques du même médicament ou de médicaments, où la quantité ou proportion dudit médicament ou desdits médicaments est la même dans les deux segments.

15. Comprimé pharmaceutique à libération immédiate tel que défini dans la revendication 6 dans lequel ledit deuxième segment est d'une couleur qui permet l'identification visuelle dudit deuxième segment en le distinguant dudit premier segment.

16. Comprimé pharmaceutique à libération immédiate tel que défini dans la revendication 6 dans lequel ledit deuxième segment contient une marque de séparation.

17. Comprimé pharmaceutique à libération immédiate tel que défini dans la revendication 16 dans lequel ledit deuxième segment contient des repères.

18. Comprimé pharmaceutique à libération immédiate tel que défini dans la revendication 17 dans lequel ledit deuxième segment contient des repères imprimés.

19. Comprimé pharmaceutique à libération immédiate tel que défini dans une quelconque des revendications 16, 17 ou 18 dans lequel la rupture dudit comprimé, lorsque l'on est guidé par une couleur, un marquage ou des repères imprimés, a pour résultat une pilule ayant une quantité prédéterminée de médicament.

20. Comprimé pharmaceutique à libération immédiate tel que défini dans la revendication 6 dans lequel les premier segment et troisième segment consistent essentiellement en un même médicament.

21. Comprimé pharmaceutique à libération immédiate tel que défini dans la revendication 10 qui est pourvu d'une marque de séparation.

22. Comprimé pharmaceutique à libération immédiate tel que défini dans les revendications 6 ou 10 où ledit deuxième segment a une couleur qui permet l'identification visuelle dudit deuxième segment en le distinguant dudit premier segment et dudit troisième segment.

23. Comprimé pharmaceutique à libération immédiate tel que défini dans les revendications 6 ou 10 dans lequel le deuxième segment correspond à un segment situé au milieu du comprimé.

24. Comprimé pharmaceutique à libération immédiate tel que défini dans la revendication 1 où ledit comprimé-noyau forme une structure de comprimé sélectionnée à partir du groupe consistant en A-I-B-I-A, A-I-B-I-B, A-I-B-I-C, ou A-B-I-C où A, B, C et I représentent des segments mutuellement différents qui sont disposés verticalement les uns sur les autres et où A, B et C contiennent un médicament ou des médicaments actif(s) et I est un segment sans quantité pharmacologiquement efficace d'un quelconque médicament.

25. Comprimé pharmaceutique à libération immédiate tel que défini dans la revendication 24 dans lequel chacun des segments A, B, C et / ou I a soit une couleur, soit des repères qui identifient chacun des segments A, B, I et C comme étant des segments différents.

26. Procédé pour rompre un comprimé pharmaceutique à libération immédiate tel que défini dans les revendications 11 ou 16 qui comprend la rupture dudit comprimé à ladite marque de séparation.

27. Comprimé pharmaceutique tel que défini dans la revendication 1 dans lequel ledit médicament ou lesdits médicaments est ou sont pharmacologiquement efficace(s) dans le traitement des maladies cardiovasculaires, des troubles psychiatriques, du diabète, des troubles de la thyroïde, de la douleur ou des troubles thrombotiques.

28. Dose partielle d'un médicament contenu dans un comprimé pharmaceutique tel que défini dans les revendications 11 ou 16, dérivée de la rupture dudit comprimé pharmaceutique à ladite marque de séparation pour former deux ou plusieurs pilules pour l'administration d'au moins une desdites pilules à un patient.
